# FASCICULE DE BREVET EUROPEEN

(11) **EP 1 855 695 B1**
(45) Date de publication et mention de la délivrance du brevet: **03.11.2010**
(21) Numéro de dépôt: 06725981.2
(22) Date de dépôt: 23.02.2006
(51) Int. Cl.: A61P 31/04, A61K 31/704, A61K 9/20

(54) **COMPOSITION PHARMACEUTIQUE SOLIDE COMPRENANT DE LA TELITHROMYCINE**
FESTE PHARMAZEUTISCHE ZUSAMMENSETZUNG MIT TELITHROMYCIN
SOLID PHARMACEUTICAL COMPOSITION COMPRISING TELITHROMYCIN

(30) Priorité: 25.02.2005 FR 0501936
(43) Date de publication de la demande: 21.11.2007
(73) Titulaire: Aventis Pharma S.A., 92160 Antony (FR)
(72) Inventeur: DESESQUELLE, Christian, F-95230 Soisy sous Montmorency (FR)
(74) Mandataire: Kugel, Dominique
(86) Numéro de dépôt international: PCT/FR2006/000411
(87) Numéro de publication internationale: WO 2006/090067

(56) Documents cités:
- FR-A- 2 826 274
- US-A1- 2004 018 737
- US-A1- 2004 091 536
- ANONYME: "ketek tablets information" INTERNET ARTICLE, [Online] 2004, XP002351340 Extrait de l'Internet: URL:http://www.drugs.com/pdr/Ketek_Tablets .html> [extrait le 2005-10-27]

## Description

L'invention concerne une nouvelle composition pharmaceutique solide de télithromycine permettant en particulier une déglutition par le patient facilitée.

La quantité unitaire de principe actif à administrer par voie orale via une composition pharmaceutique solide impose souvent de préparer des comprimés d'une taille telle que leur déglutition peut poser des difficultés à beaucoup de patients, aux enfants bien sûr, mais pas seulement. En outre, la nature du principe actif impose d'y adjoindre un certain nombre d'excipients parfois eux-mêmes en quantité importante afin d'assurer la cohésion de la formulation, mais aussi d'assurer une fonction importante, voire essentielle, par exemple lorsqu'un masquage du goût du principe actif est recherché. Ceci est tout particulièrement le cas pour les composés de type macrolides/kétolides comme la télithromycine.

Le principe actif télithromycine, ou 11,12-dideoxy-3-de((2,6-dideoxy-3-C-méthyl-3-O-méthyl-alpha-L-ribohexopyranosyl)oxy)-6-O-méthyl-3-oxo-12,11-(oxycarbonyl((4-(4-(3-pyridinyl)-1H-imidazol-1-yl)butyl)imino))-érythromycine, est décrit dans le brevet européen EP 0 680 967. La voie orale est une forme d'administration privilégiée pour ce principe actif doué de propriétés antibiotiques.

Les documents US 2004/0013737, FR2 826 274 et US 2004/0091536 décrivent des formulations pharmaceutiques de télithromycine sous forme de poudre (atomisat, granules) destinées à l'administation en suspension aqueuse par voie orale.

Des comprimés de télithromycine dosés à 400 mg et 300 mg sont actuellement commercialisés. Ces comprimés renferment une proportion de principe actif de 50% en poids, par rapport au poids du comprimé avant enrobage, ainsi qu'une proportion importante d'excipient permettant la granulation (près de 30 % en poids de lactose), un mélange d'excipients permettant la désintégration (près de 13 % en poids d'un mélange d'amidon de maïs, très majoritaire, et de croscarmellose sodique, très minoritaire), un faible pourcentage d'agent diluant (entre 4 et 5 % en poids de cellulose microcristalline), ainsi qu'un faible pourcentage de liant (moins de 2 % en poids) et de lubrifiant (moins de 1 % en poids), ces comprimés étant recouverts d'un pelliculage.

Ces comprimés à 400 mg et 300 mg de télithromycine présentent ainsi une masse totale respectivement de 800 et 600 mg (non compris le pelliculage) et ont une forme oblongue aux dimensions importantes, respectivement de (18 mm x 9 mm) et (13,9 mm x 8,7 mm). On conçoit donc aisément que la déglutition de tels comprimés par les patients soit parfois difficile.

Il était donc souhaitable de parvenir à réduire la taille de ces comprimés de télithromycine.

On peut avoir l'idée de résoudre ce problème simplement en augmentant la proportion de principe actif dans la composition du comprimé et donc, à dosage égal en principe actif, d'en réduire la taille. Toutefois, le principe actif télithromycine présente des propriétés mécaniques spécifiques telles que ceci n'est pas possible pour les comprimés actuellement commercialisés : la proportion de 50 % en poids de télithromycine dans les compositions avant enrobage pour les comprimés commercialisés, tel qu'indiqué plus haut, constitue un maximum.

Par ailleurs, outre ce problème de taille des comprimés, inhérent à ce principe actif particulier, il existe un autre inconvénient lié à la télithromycine se manifestant lors de la mise en oeuvre du processus industriel de préparation des comprimés.

La télithromycine est en effet un principe actif dont la compression est difficile. La conséquence est que les caractéristiques mécaniques des comprimés obtenus ne sont pas satisfaisantes, notamment pour permettre une production à haut débit. Lors du processus de compression, une tendance au clivage des comprimés peut apparaître, ce qui entraîne un taux de rejet important de comprimés déclarés non conformes pour défaut d'aspect lié à un manque de cohésion et, par conséquent, une perte économique notable.

On a maintenant trouvé de manière tout à fait surprenante et inattendue une nouvelle composition pour des comprimés de télithromycine grâce à laquelle il est possible de remédier simultanément aux inconvénients majeurs mentionnés ci-dessus, en particulier, d'une part, de pouvoir inclure une proportion très supérieure du principe actif télithromycine, allant jusqu'à 80 % en poids du comprimé hors pelliculage et, par conséquent, à dosage égal en principe actif, de fournir des comprimés dont la taille est très sensiblement réduite et, d'autre part, de pouvoir augmenter de manière tout à fait significative la résistance à la rupture du comprimé avant enrobage et, par conséquent, de réduire très sensiblement à l'échelle industrielle le taux de rejet de comprimés pour défaut d'aspect lié à un manque de cohésion.

La présente invention a ainsi pour objet un comprimé comprenant de la télithromycine ou un de ses sels d'addition avec un acide pharmaceutiquement acceptable, en tant que principe actif, **caractérisé en ce qu**'il comprend, par rapport au poids total de la composition :
- de la télithromycine ou un de ses sels d'addition avec un acide pharmaceutiquement acceptable, selon une proportion en télithromycine comprise entre 0,1 et 80 % en poids, et
- au moins un agent diluant à comportement plastique, qui est de la cellulose microcristalline selon une proportion de 10 à 50 % en poids.

A moins qu'il ne le soit précisé autrement, les proportions indiquées en poids par rapport au poids total de la composition s'entendent par rapport au poids total de la composition sans compter un enrobage éventuel de cette dernière notamment par un pelliculage.

Plus particulièrement, les proportions en poids indiquées pour le principe actif télithromycine, qu'il soit présent ou non sous forme d'un de ses sels d'addition avec un acide pharmaceutiquement acceptable, sont des proportions calculées en poids du composé télithromycine par rapport au poids total de la composition sans compter un enrobage éventuel de cette dernière notamment par un pelliculage.

Comme sel d'addition possible de la télithromycine avec un acide pharmaceutiquement acceptable, on peut citer en particulier les sels d'addition avec les acides minéraux ou organiques notamment les sels formés avec les acides acétique, propionique, trifluoroacétique, maléique, tartrique, méthanesulfonique, benzènesulfonique, p-toluènesulfonique, chlorhydrique, bromhydrique, iodhydrique, sulfurique, phosphorique et plus particulièrement les acides stéarique, éthylsuccinique ou laurylsulfurique.

La composition selon l'invention comprend de préférence de la télithromycine ou un de ses sels d'addition avec un acide pharmaceutiquement acceptable, selon une proportion en télithromycine comprise entre 50 et 80 % en poids, plus particulièrement entre 60 et 80 % en poids et plus particulièrement encore entre 60 et 70 % en poids, par rapport au poids total de la composition.

Selon la présente invention, on a en particulier constaté de manière surprenante qu'en augmentant très sensiblement la proportion d'un des excipients dans la composition déjà commercialisée pour des comprimés de télithromycine, indiquée ci-dessus, au détriment de deux autres excipients particuliers de cette même composition déjà connue, il est possible à la fois d'augmenter la proportion du principe actif télithromycine, jusqu'à au moins 80 % en poids, tout en améliorant la résistance à la rupture des comprimés notamment lors du processus de compression de la composition. Sans vouloir être tenu à une quelconque théorie, il apparaît que l'augmentation très sensible de la proportion de cet excipient particulier, à savoir l'agent diluant, qui est la cellulose microcristalline, au détriment pourtant de deux autres excipients particuliers déjà présents dans cette même composition déjà connue, à savoir le lactose et l'amidon de maïs, permettrait de conférer à cet agent diluant une fonction plastifiante déterminante pour les propriétés mécaniques de la composition de télithromycine, notamment lors de sa compression, d'où cette expression « agent diluant à comportement plastique ».

On a par ailleurs pu constater que ce résultat aussi intéressant qu'inattendu est encore amélioré en agissant sur les autres excipients également présents dans la composition des comprimés commercialisés.

Ainsi, de préférence, le comprimé selon l'invention comprend en outre, par rapport au poids total de la composition :
- au moins un agent liant, selon une proportion de 2,5 à 3,5 % en poids,
- au moins un agent désintégrant, selon une proportion de 3 à 8 % en poids, et
- au moins un agent lubrifiant, selon une proportion de 0,6 à 1 % en poids.

De préférence, l'agent diluant à comportement plastique à savoir la cellulose microcristalline, est présent selon une proportion comprise entre 20 et 30 % en poids, par rapport au poids total de la composition selon l'invention.

Plus particulièrement, le comprimé selon la présente invention est caractérisé en ce qu'il comprend, par rapport au poids total de la composition :
- de la télithromycine ou un de ses sels d'addition avec un acide pharmaceutiquement acceptable, selon une proportion en télithromycine comprise entre 50 et 80 % en poids, plus particulièrement entre 60 et 80 % en poids et plus particulièrement encore entre 60 et 70 % en poids,
- au moins un agent diluant à comportement plastique, à savoir la cellulose microcristalline, selon une proportion de 20 à 30 % en poids,
- au moins un agent liant, selon une proportion de 2,8 à 3 % en poids,
- au moins un agent désintégrant, selon une proportion de 3,5 à 6 % en poids, et
- au moins un agent lubrifiant, selon une proportion de 0,6 à 1% en poids.

Selon un mode particulier de réalisation, le comprimé selon l'invention est **caractérisé en ce que** :
- l'agent liant est choisi dans le groupe constitué par la povidone K25, la povidone K30, la copovidone, l'hydroxypropyl cellulose et les mélanges de ceux-ci,
- l'agent désintégrant est choisi dans le groupe constitué par la croscarmellose sodique, la crospovidone, le carboxyméthylamidon sodique et les mélanges de ceux-ci,
- l'agent lubrifiant est choisi dans le groupe constitué par le stéarate de magnésium, l'acide stéarique micronisé et les mélanges de ceux-ci.

Plus particulièrement encore, le comprimé selon l'invention est **caractérisé en ce que** :
- l'agent liant est choisi dans le groupe constitué par la povidone K25, la povidone K30 et les mélanges de ceux-ci,
- l'agent désintégrant est la croscarmellose sodique, et
- l'agent lubrifiant est le stéarate de magnésium.

Enfin, selon un mode tout particulièrement préféré de réalisation, le comprimé selon l'invention est **caractérisé en ce qu'**il comprend, par rapport au poids total de la composition :
- de 60 à 70 % en poids de télithromycine,
- de 20 à 30 % en poids de cellulose microcristalline,
- de 2,8 à 3 % en poids de povidone choisie dans le groupe constitué par la povidone K25, la povidone K30 et les mélanges de ceux-ci,
- de 3,5 à 6 % en poids de croscarmellose sodique, et
- de 0,6 à 1% en poids de stéarate de magnésium.

La télithromycine peut en particulier être présente dans le comprimé selon l'invention sous forme de granulats obtenus par granulation, notamment par granulation humide : le comprimé selon l'invention comprenant ainsi des granulats de télithromycine dispersés dans une phase externe comprenant d'autres composants de la composition, avant ou après le processus de compression. En particulier, les granulats de télithromycine comprennent en outre ledit agent liant et la phase externe comprend lesdits agents désintégrant et lubrifiant. On a pu observer que l'agent diluant à comportement plastique peut être présent dans lesdits granulats de télithromycine et ladite phase externe.

Le comprimé peut encore être soumis en particulier à une opération d'enrobage, notamment par un pelliculage selon des conditions opératoires bien connues de l'homme du métier. Ainsi, en particulier, le comprimé selon l'invention est **caractérisé en ce qu'**il est recouvert d'un pelliculage. Plus particulièrement, ce pelliculage peut comprendre au moins un composant choisi dans le groupe constitué par l'hypromellose, le polyéthylène glycol, le dioxyde de titane, le talc, l'oxyde de fer jaune, l'oxyde de fer rouge et les mélanges de ceux-ci.

Enfin, le comprimé selon l'invention comprend de préférence de 50 mg à 600 mg de télithromycine, en particulier 400 mg ou encore 300 mg de télithromycine.

Le comprimé selon l'invention permet ainsi, pour des dosages de principe actif télithromycine de 400 et 300 mg, c'est-à-dire identique à celui des comprimés existants, d'avoir des dimensions réduites. Les comprimés selon l'invention ont des tailles qui sont respectivement de (13,9 mm x 8,7 mm) et (12,5 x 7,8 mm).

Au final, le comprimé selon l'invention peut donc avantageusement présenter une taille réduite, à dosage pourtant égal en télithromycine, par rapport aux comprimés de la composition commercialisée, ce qui présente en soi un avantage très important tant pour l'observance par les patients que pour la réduction des coûts de production du produit fini pharmaceutique. Qui plus est, comme indiqué dans l'étude ci-après, ces comprimés à taille réduite présentent une meilleure dureté ce qui, à l'échelle industrielle, se traduit par une diminution très avantageuse du taux de rejet de comprimés pour défaut d'aspect lié à un manque de cohésion, par exemple de 2% à 0,02% pour des comprimés à 300 mg de télithromycine.

Les exemples suivants sont destinés à illustrer la présente invention et ne doivent en aucun cas être interprétés comme pouvant en limiter la portée.

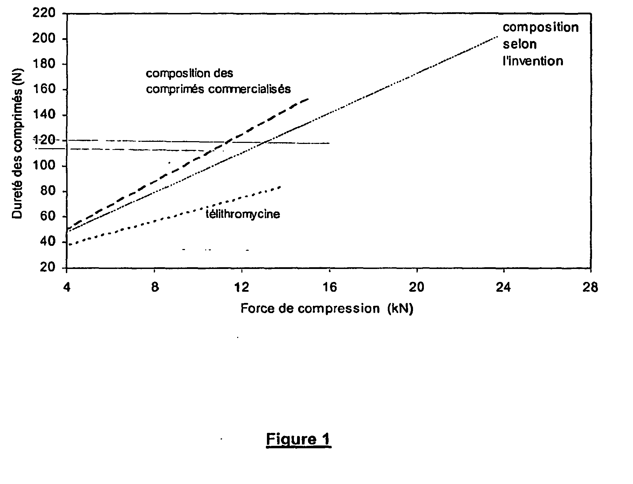

La Figure 1 représente trois courbes en pointillés montrant la dureté (en N) de trois comprimés en fonction de la force de compression (en kN) selon le test de résistance à la rupture décrit ci-après. La courbe en pointillés les plus petits correspond au test sur la composition selon l'invention préparée à l'exemple 1.1 (avant compression et pelliculage), la courbe en pointillés les plus grands correspond au test sur la composition des comprimés commercialisés (avant compression et pelliculage), et la courbe en pointillés intermédiaires en taille correspond au test sur la poudre de principe actif télithromycine.

### Exemple 1 : comprimés à 400 mg de télithromycine

On réalise des comprimés à base de télithromycine dont la composition est reportée dans le Tableau 1 suivant (composition selon l'invention, recouverte d'un pelliculage).

On opère pour cela de la manière suivante.

### 1.1 Préparation du comprimé

On introduit dans un mélangeur granulateur et mélange pendant 5 minutes, 133,00 kg de télithromycine et 39,20 kg de cellulose microcristalline (Avicel PH 101).

On mélange séparément 60,60 kg d'eau purifiée et 6,00 kg de Povidone K25, puis verse la solution limpide obtenue sur le mélange de poudres obtenu plus haut maintenu sous agitation.

On émotte les granulats humides ainsi obtenus à l'aide d'un calibreur rotatif CoMil équipé d'une grille de 9,5 mm d'ouverture de maille, puis transfère les granulats émottés dans une cuve de séchage. On les y maintient à 60 °C dans un sécheur à lit d'air fluidisé jusqu'à obtenir une solvatation résiduelle inférieure à 2%.

On tamise ensuite les granulats séchés à l'aide d'un calibreur équipé d'une grille de 1,6 mm d'ouverture de maille.

On ajoute aux granulats calibrés 12,000 kg de cellulose microcristalline (Avicel PH 101) et 7,980 kg de croscarmellose sodique puis maintient sans agitation pendant 10 minutes.

On ajoute ensuite 1,320 kg de stéarate de magnésium puis maintient son agitation pendant 4 minutes.

On comprime ensuite les granulats ainsi obtenus à l'aide d'une presse à comprimer équipée de l'outillage nécessaire pour le dosage souhaité de 400 mg.

On obtient ainsi des comprimés de composition indiquée au tableau 1 (sans le pelliculage) dont la taille est de (13,9 mm x 8,7 mm) au lieu de (18 mm x 9 mm) pour les comprimés commercialisés.

### 2.2 Pelliculage du comprimé

On prépare une solution de pelliculage comme suit.

Dans 133,5 kg d'eau purifiée, on introduit lentement sous agitation les composants suivants, un à un :
- 24,000 kg d'hydroxypropyl méthylcellulose 6 cP (hypromellose 6 cp),
- 1,050 kg de polyéthylène glycol 8000,
- 1,800 kg de talc,
- 5,100 kg de dioxyde de titane,
- 0,750 kg d'oxyde de fer jaune,
- 0,150 kg d'oxyde de fer rouge,
puis maintient sous agitation jusqu'à obtention d'une suspension homogène.

On rajoute ensuite 133,5 kg d'eau purifiée puis continue l'agitation pendant 5 minutes.

La suspension obtenue est utilisée pour pelliculer les comprimés obtenus plus haut, dans une turbine à pelliculer de type Accela Cota.

**Tableau 1 : composition pour comprimés pelliculés de télithromycine**

| **Composant** | **Proportion** |
|---|---|
| | **(% en poids ; avec pelliculage)** |
| **Principe actif** | |
| | 65.20 % |
| télithromycine | (400 mg ) |
| **Excipients** | |
| cellulose microcristalline | 25.10 % |
| croscarmellose sodique ² | 3.91 % |
| povidone K25 ³ | 2.93% |
| stéarate de magnesium | 0.65 % |
| **Pelliculage** | |
| hypromellose 6 cP ⁴ | 1.62 % |
| polyethylene glycol 8000 ⁵ | 0.07 % |
| dioxyde de Titane | 0.34 % |
| Talc | 0.12 % |
| oxyde de fer jaune | 0.05 % |
| oxyde de fer rouge | 0.01 % |
| **Total (avec pelliculage)** | 100.00 % |

| | |
|---|---|
| ¹ : Avicel PH 1001 commercialisé par FMC ² : Ac-Di-Sol commercialisé par FMC ³ : Kollidon 25 commercialisé par BASF ⁴ : Pharmacoat 606 commercialisé par Shin-Etsu ⁵ : Polyglykol 8000P commercialisé par Hoechst | |

### Etude de la résistance à la rupture de la composition selon l'invention sous forme de comprimé

La comprimabilité d'une poudre est évaluée par sa capacité à former un compact lorsqu'elle est soumise à une pression. Ceci est réalisé dans le cadre du procédé illustré ci-dessus, en utilisant une presse à comprimer alternative équipée de poinçons porteurs de jauges de contrainte. Un système d'amplification et un convertisseur permettent à tout moment d'enregistrer les forces appliquées durant le cycle de compression. A la fin du processus, la résistance à la rupture du comprimé formé est mesurée par un banc de dureté. On obtient ainsi une mesure de résistance à la rupture ou « dureté » du comprimé (en N) pour une force de compression donnée (en kN). Pour une poudre à tester, on répète ce processus plusieurs fois de manière à pouvoir tracer une courbe telle que celles de la Figure 1.

Des essais comparatifs ont ainsi été réalisés sur la poudre du principe actif télithromycine, sur la composition utilisée pour la fabrication des comprimés commercialisés, avant compression et pelliculage (à 50% en poids de télithromycine hors pelliculage) et sur la composition selon l'invention préparée à l'exemple 1.1 ci-dessus, avant compression et pelliculage (à 66,7% en poids de télithromycine hors pelliculage).

Les résultats de ces essais comparatifs sont reportés sous la forme des trois courbes respectives à la Figure 1.

Ces essais comparatifs montrent l'amélioration apportée par la composition selon l'invention, illustrée par la différence de longueur des courbes qui témoignent de l'augmentation de la cohésion des comprimés proportionnellement à la pression exercée. Au-delà d'une courbe, les duretés des comprimés stagnent ou diminuent, indiquant des phénomènes de clivage.

A l'échelle industrielle, sur des comprimés similaires mais dosés à 300 mg de télithromycine, on a pu constater que cette amélioration se traduit par une réduction très avantageuse de 2 % à 0,02 % du taux de rejet de comprimés pour défaut d'aspect lié à un manque de cohésion.

## Revendications

1. Comprimé comprenant de la télithromycine ou un de ses sels d'addition avec un acide pharmaceutiquement acceptable, en tant que principe actif, **caractérisé en ce qu'**il comprend, par rapport au poids total de la composition :
- de la télithromycine ou un de ses sels d'addition avec un acide pharmaceutiquement acceptable, selon une proportion en télithromycine comprise entre 0,1 et 80 % en poids, et
- au moins un agent diluant à comportement plastique, qui est de la cellulose microcristalline, selon une proportion de 10 à 50 % en poids.

2. Comprimé selon la revendication 1, **caractérisé en ce qu'**il comprend en outre, par rapport au poids total de la composition :
- au moins un agent liant, selon une proportion de 2,5 à 3,5 % en poids,
- au moins un agent désintégrant, selon une proportion de 3 à 8 % en poids, et
- au moins un agent lubrifiant, selon une proportion de 0,6 à 1 % en poids.

3. Comprimé selon la revendication 1 ou 2, **caractérisé en ce qu'**il comprend, par rapport au poids total de la composition :
- de la télithromycine ou un de ses sels d'addition avec un acide pharmaceutiquement acceptable, selon une proportion en télithromycine comprise entre 60 et 70 % en poids,
- au moins un agent diluant à comportement plastique, qui est la cellulose microcristalline selon une proportion de 20 à 30 % en poids,
- au moins un agent liant, selon une proportion de 2,8 à 3 % en poids,
- au moins un agent désintégrant, selon une proportion de 3,5 à 6 % en poids, et
- au moins un agent lubrifiant, selon une proportion de 0,6 à 1% en poids

4. Comprimé selon la revendication 2 ou 3, **caractérisé en ce que** :
- l'agent liant est choisi dans le groupe constitué par la povidone K25, la povidone K30, la copovidone, l'hydroxypropyl cellulose et les mélanges de ceux-ci,
- l'agent désintégrant est choisi dans le groupe constitué par la croscarmellose sodique, la crospovidone, le carboxyméthylamidon sodique et les mélanges de ceux-ci,
- l'agent lubrifiant est choisi dans le groupe constitué par le stéarate de magnésium, l'acide stéarique micronisé et les mélanges de ceux-ci.

5. Comprimé selon la revendication 2 ou 3, **caractérisé en ce que** :
- l'agent liant est choisi dans le groupe constitué par la povidone K25, la povidone K30 et les mélanges de ceux-ci,
- l'agent désintégrant est la croscarmellose sodique, et
- l'agent lubrifiant est le stéarate de magnésium.

6. Comprimé selon l'une quelconque des revendications précédentes, **caractérisé en ce qu'**il comprend, par rapport au poids total de la composition :
- de 60 à 70 % en poids de télithromycine,
- de 20 à 30 % en poids de cellulose microcristalline,
- de 2,8 à 3 % en poids de povidone choisie dans le groupe constitué par la povidone K25, la povidone K30 et les mélanges de ceux-ci,
- de 3,5 à 6 % en poids de croscarmellose sodique, et
- de 0,6 à 1% en poids de stéarate de magnésium.

7. Comprimé pharmaceutique selon l'une quelconque des revendications précédentes, **caractérisé en ce qu'**il est recouvert d'un pelliculage.

8. Comprimé pharmaceutique selon la revendication 7, **caractérisé en ce que** ledit pelliculage comprend au moins un composant choisi dans le groupe constitué par l'hypromellose, le polyéthylène glycol, le dioxyde de titane, le talc, l'oxyde de fer jaune, l'oxyde de fer rouge et les mélanges de ceux-ci.

9. Comprimé selon l'une quelconque des revendications précédentes, **caractérisé en ce qu'**il comprend de 50 mg à 600 mg de télithromycine.

10. Comprimé selon l'une quelconque des revendications précédentes, **caractérisé en ce qu'**il comprend 400 mg de télithromycine.

11. Comprimé selon l'une quelconque des revendications précédentes, **caractérisé en ce qu'**il comprend 300 mg de télithromycine.

## Claims

1. Tablet comprising telithromycin or an addition salt thereof with a pharmaceutically acceptable acid, as an active ingredient, **characterized in that** it comprises, relative to the total weight of the composition:
- telithromycin, or an addition salt thereof with a pharmaceutically acceptable acid, in a proportion of telithromycin of between 0.1 and 80% by weight, and
- at least one diluent having a plastic behaviour, which is microcrystalline cellulose, in a proportion of 10 to 50% by weight.

2. Tablet according to Claim 1, **characterized in that** it also comprises, relative to the total weight of the composition:
- at least one binder, in a proportion of 2.5 to 3.5% by weight,
- at least one disintegrating agent, in a proportion of 3 to 8% by weight, and
- at least one lubricant, in a proportion of 0.6 to 1% by weight.

3. Tablet according to Claim 1 or 2, **characterized in that** it comprises, relative to the total weight of the composition:
- telithromycin, or an addition salt thereof with a pharmaceutically acceptable acid, in a proportion of telithromycin of between 60 and 70% by weight,
- at least one diluent having a plastic behaviour, which is microcrystalline cellulose, in a proportion of 20 to 30% by weight,
- at least one binder, in a proportion of 2.8 to 3% by weight,
- at least one disintegrating agent, in a proportion of 3.5 to 6% by weight, and
- at least one lubricant, in a proportion of 0.6 to 1% by weight.

4. Tablet according to Claim 2 or 3, **characterized in that**:
- the binder is chosen from the group consisting of povidone K25, povidone K30, copovidone and hydroxypropylcellulose, and mixtures thereof,
- the disintegrating agent is chosen from the group consisting of sodium croscarmellose, crospovidone and sodium carboxymethyl starch, and mixtures thereof,
- the lubricant is chosen from the group consisting of magnesium stearate and micronized stearic acid, and mixtures thereof.

5. Tablet according to Claim 2 or 3, **characterized in that**:
- the binder is chosen from the group consisting of povidone K25 and povidone K30, and mixtures thereof,
- the disintegrating agent is sodium croscarmellose, and
- the lubricant is magnesium stearate.

6. Tablet according to any one of the preceding claims, **characterized in that** it comprises, relative to the total weight of the composition:
- from 60 to 70% by weight of telithromycin,
- from 20 to 30% by weight of microcrystalline cellulose,
- from 2.8 to 3% by weight of povidone chosen from the group consisting of povidone K25 and povidone K30, and mixtures thereof,
- from 3.5 to 6% by weight of sodium croscarmellose, and
- from 0.6 to 1% by weight of magnesium stearate.

7. Pharmaceutical tablet according to any one of the preceding claims, **characterized in that** it is coated with a film-coating.

8. Pharmaceutical tablet according to Claim 7, **characterized in that** said film-coating comprises at least one component chosen from the group consisting of hypromellose, polyethylene glycol, titanium dioxide, talc, yellow iron oxide and red iron oxide, and mixtures thereof.

9. Tablet according to any one of the preceding claims, **characterized in that** it comprises from 50 mg to 600 mg of telithromycin.

10. Tablet according to any one of the preceding claims, **characterized in that** it comprises 400 mg of telithromycin.

11. Tablet according to any one of the preceding claims, **characterized in that** it comprises 300 mg of telithromycin.

## Patentansprüche

1. Tablette, enthaltend Telithromycin oder eines seiner Additionssalze mit einer pharmazeutisch zulässigen Säure als Wirkstoff, **dadurch gekennzeichnet, dass** sie, bezogen auf das Gesamtgewicht der Zusammensetzung, Folgendes umfasst:
- Telithromycin oder eines seiner Additionssalze mit einer pharmazeutisch zulässigen Säure, wobei der Anteil an Telithromycin zwischen 0,1 und 80 Gew.-% umfasst, und
- mindestens ein Verdünnungsmittel mit plastischem Verhalten, welches mikrokristalline Cellulose ist, wobei der Anteil 10 bis 50 Gew.-% umfasst,

2. Tablette nach Anspruch 1, **dadurch gekennzeichnet, dass** sie des Weiteren, bezogen auf das Gesamtgewicht der Zusammensetzung, Folgendes umfasst:
- mindestens ein Bindemittel, wobei der Anteil 2,5 bis 3,5 Gew.-% umfasst,
- - mindestens ein Sprengmittel, wobei der Anteil 3 bis 8 Gew.-% umfasst, und
- mindestens ein Schmiermittel, wobei der Anteil 0, 6 bis 1 Gew.-% umfasst.

3. Tablette nach Anspruch 1 oder 2, **dadurch gekennzeichnet, dass** sie des Weiteren, bezogen auf das Gesamtgewicht der Zusammensetzung, Folgendes umfasst:
- Telithromycin oder eines seiner Additionssalze mit einer pharmazeutisch zulässigen Säure, wobei der Anteil an Telithromycin zwischen 60 und 70 Gew.-% umfasst,
- mindestens ein Verdünnungsmittel mit plastischem Verhalten, welches mikrokristalline Cellulose ist, wobei der Anteil 20 bis 30 Gew.-% umfasst,
- - mindestens ein Bindemittel, wobei der Anteil 2, 8 bis 3 Gew.-% umfasst,
- mindestens ein Sprengmittel, wobei der Anteil 3, 5 bis 6 Gew.-% umfasst, und
- mindestens ein Schmiermittel, wobei der Anteil 0, 6 bis 1 Gew.-% umfasst.

4. Tablette nach Anspruch 2 oder 3, **dadurch gekennzeichnet, dass**:
- das Bindemittel aus der Gruppe bestehend aus Povidon K25, Povidon K30, Copovidon, Hydroxypropylcellulose sowie deren Mischungen ausgewählt wird,
- das Sprengmittel aus der Gruppe bestehend aus Croscarmellose-Natrium, Crospovidon, Carboxymethylamidon-Natrium sowie deren Mischungen ausgewählt wird,
- das Schmiermittel aus der Gruppe bestehend aus Magnesiumstearat, mikronisierter Stearinsäure sowie deren Mischungen ausgewählt wird.

5. Tablette nach Anspruch 2 oder 3, **dadurch gekennzeichnet, dass**:
- das Bindemittel aus der Gruppe bestehend aus Povidon K25, Povidon K30 sowie deren Mischungen ausgewählt wird,
- das Sprengmittel Croscarmellose-Natrium ist, und
- das Schmiermittel Magnesiumstearat ist.

6. Tablette nach einem der vorhergehenden Ansprüche, **dadurch gekennzeichnet, dass** sie, bezogen auf das Gesamtgewicht der Zusammensetzung, Folgendes umfasst:
- - 60 bis 70 Gew.-% Telithromycin,
- 20 bis 30 Gew.-% mikrokristalline Cellulose,
- 2,8 bis 3 Gew.-% Povidon, das aus der Gruppe bestehend aus Povidon K25, Povidon K30 sowie deren Mischungen ausgewählt wird,
- 3,5 bis 6 Gew.-% Croscarmellose-Natrium, und
- 0,6 bis 1 Gew.-% Magnesiumstearat.

7. Pharmazeutische Tablette nach einem der vorhergehenden Ansprüche, **dadurch gekennzeichnet, dass** sie mit einer Filmbeschichtung überzogen ist.

8. Pharmazeutische Tablette nach Anspruch 7, **dadurch gekennzeichnet, dass** diese Filmbeschichtung zumindest aus einem Bestandteil besteht, der aus der Gruppe bestehend aus Hypromellose, Polyethylenglykol, Titandioxid, Talkum, gelbem Eisenoxid, rotem Eisenoxid und Mischungen daraus ausgewählt wird.

9. Tablette nach einem der vorhergehenden Ansprüche, **dadurch gekennzeichnet, dass** sie 50 mg bis 600 mg Telithromycin enthält.

10. Tablette nach einem der vorhergehenden Ansprüche, **dadurch gekennzeichnet, dass** sie 400 mg Telithromycin enthält.

11. Tablette nach einem der vorhergehenden Ansprüche, **dadurch gekennzeichnet, dass** sie 300 mg Telithromycin enthält.
